# EUROPÄISCHE PATENTSCHRIFT

(11) **EP 3 033 051 B1**
(45) Veröffentlichungstag und Bekanntmachung des Hinweises auf die Patenterteilung: **12.07.2017**
(21) Anmeldenummer: 14753205.5
(22) Anmeldetag: 12.08.2014
(51) Int. Cl.: A61F 2/78, A61F 2/80

(54) **PROTHESENSCHAFT MIT EINFÜHRHILFSEINRICHTUNG**
PROSTHESIS STEM HAVING AN INSERTION AID APPARATUS
EMBOÎTURE DE PROTHÈSE DOTÉE D'UN DISPOSITIF D'AIDE À L'INTRODUCTION

(30) Priorität: 12.08.2013 DE 102013013314
(43) Veröffentlichungstag der Anmeldung: 22.06.2016
(73) Patentinhaber: Otto Bock HealthCare GmbH, 37115 Duderstadt (DE)
(72) Erfinder: RADSPIELER, Andreas, 83115 Neubeuern (DE)
(74) Vertreter: Stornebel, Kai
(86) Internationale Anmeldenummer: PCT/EP2014/002207
(87) Internationale Veröffentlichungsnummer: WO 2015/022069

(56) Entgegenhaltungen:
- WO-A1-2009/062489
- WO-A2-2007/107800
- WO-A2-2014/075808
- GB-A- 1 086 560
- US-A- 5 211 667
- US-A1- 2005 240 283
- US-A1- 2010 249 950

## Beschreibung

Die Erfindung betrifft einen Prothesenschaft mit einem Schaftkörper zur Aufnahme eines Amputationsstumpfs und einer Einführhilfseinrichtung, die das Einführen des Amputationsstumpfs in den Schaftkörper erleichtert.

Bei Arm- und Beinprothesen sind verschiedene Möglichkeiten bekannt, die Prothese an- und auszuziehen. Bei Linersystemen wird ein Liner (Kompressionsstrumpf) zunächst auf links gewendet und über den Amputationsstumpf gezogen oder auf diesen aufgerollt. Dies erfolgt zunächst völlig unabhängig und getrennt vom Prothesenschaft. Der Liner sitzt dabei derart eng am Amputationsstumpf an, dass eine Kompression des Amputationsstumpfes erreicht wird. Weiterhin können derartige Liner an ihrem distalen Ende mit einem Fixierstift versehen sein, um den Liner, nachdem der Amputationsstumpf in den Prothesenschaft eingesetzt worden ist, am Prothesenschaft zu verriegeln. Aufgrund der Gasdichtheit und Reibungshaftung sitzt der Liner fest am Amputationsstumpf.

Ein Prothesenschaft dieser Art ist aus der WO 2009/062489 A1, auf der der Oberbegriff des Anspruchs 1 basiert, bekannt. Der dortige Prothesenschaft kann darüber hinaus auch einen Anschluss im Schaftkörper aufweisen, um eine Vakuumpumpe anzuschließen und dadurch das Vakuum zwischen Schaftkörper und Liner zu erhöhen.

Die DE 10 2011 105 488 A1 betrifft eine Vorrichtung zum Befestigen einer Körperprothese an intakten Körperteilen mittels Unterdruck zwischen einer über den Stumpf gezogenen Linereinheit und einen den Stumpf umschließenden Prothesenschaft. Die Linereinheit sieht einen dem Stumpf zugewandten ersten Liner und einen außen daran angeordneten zweiten Liner vor, der über den Rand des Prothesenschaftes hinausragt. Der hinausragende Bereich des zweiten Liners ist so gestaltet, dass er nach dem Umschlagen über den Rand des Prothesenschaftes an der Außenseite des Prothesenschaftes haftet und eine luftdichte Verbindung herstellt.

Die DE 202 17 525 U1 betrifft ein Mittel zum Anziehen beispielsweise von Prothesenschäften, das zumindest zwei ineinanderliegende Hüllen aufweist, wobei die innere Hülle zum Anziehen des Prothesenschaftes in die äußere Hülle gestülpt ist. Beide Hüllen sind an einem ersten Ende miteinander verbunden. An dem zweiten Ende sind Greifmittel an der inneren Hülle vorgesehen, die äußere Hülle weist einen Durchgang auf, durch die die Greifmittel von außen erreichbar sind.

Die DE 10 2012 022 414 A1 betrifft einen Prothesenschaft mit einem Schaftkörper, in den ein Stumpf einführbar ist, und mit einem flexiblen Inlay, das bei einem angelegten Prothesenschaft zwischen dem Schaftkörper und dem Stumpf angeordnet ist. Das Inlay begrenzt zusammen mit dem Schaftkörper einen Fluiddruckraum, der mit einer Überdruckerzeugungseinrichtung verbunden und derart luftdicht ausgebildet ist, dass durch Erzeugung eines Überdruckes in dem Fluiddruckraum auf einen Endbereich des Inlays eine Verschiebekraft in proximaler Richtung ausgeübt wird, um das Ablegen des Prothesenschaftes zu erleichtern.

Bei Linersystemen ist es weiterhin bekannt, am distalen Ende des Liners anstelle eines Fixierstiftes eine Einführhilfseinrichtung in der Form einer Kordel zu befestigen, die durch eine Öffnung im Schaftboden hindurch gezogen wird und mittels einer Seilklemme fixiert werden kann. Zum Ausziehen der Prothese wird der Rastmechanismus des Verriegelungsstiftes beziehungsweise die Seilklemme entriegelt und der Prothesenschaft vom Stumpf abgezogen.

Nachteilig ist bei den bekannten Prothesenschäften jedoch, dass das An- und Ausziehen der Prothese nur mit hohem Kraftaufwand möglich ist. Um einen Prothesenschaft optimal zu nutzen, sollte dieser möglichst eng am Amputationsstumpf sitzen. Je enger der Amputationsstumpf am Prothesenschaft sitzt, umso schwieriger ist jedoch das An- und Ausziehen der Prothese. Liner erleichtern zwar das Einführen des Amputationsstumpfs in den Prothesenschaft, haben jedoch den Nachteil, dass sie erst mit hohem Kraftaufwand über den Amputationsstumpf gezogen werden müssen.

Dies ist insbesondere für schwache, ältere und verletzte Personen sowie für Personen, die einen amputierten Arm haben, mit ganz erheblichen Problemen verbunden.

Die GB 1,086,560 A betrifft eine Verbesserung hinsichtlich der prothetischen Versorgung von Patienten mit einem ersten Strumpf oder einer ersten Hülse aus einem Polyamidgarn, das auf seiner Außenseite mit einer dickeren Textilschicht aus Wolle versehen ist. Sowohl die Polyamidhülse als auch die Wollschicht sind über Knöpfe oder Klettverschlüsse an der Außenseite des Prothesenschaftes befestigt.

Die WO 2007/107800 A2 betrifft eine Prothesenschafteinrichtung mit einem stabilen Schaft und einer darin angeordneten Stumpfaufnahme, die über ein im Schaft befindliches Federsystem elastisch gehalten ist. Die Stumpfaufnahme kann Blattfedern aufweisen, die sich an den Stumpf anlegen.

Die US 2005/0240283 A1 betrifft einen Prothesenschaft mit unterschiedlichen Wandstärken und einem gegebenenfalls distalen Ende angeordneten Verstärkungseinsatz. Der Prothesenliner kann unterschiedliche Materialien oder Bereiche mit unterschiedlichen mechanischen Eigenschaften aufweisen.

Die US 5,211,667 A betrifft ein Prothesensystem mit einem teleskopierbaren Prothesenschaft und einem darin angeordneten Liner. Der Prothesenschaft weist eine distale, geschlossene Hülse sowie eine proximale offene Hülse auf, die gegeneinander verschieblich sind und über Bänder mit Klettverschlüssen in der gewünschten Position zueinander fixierbar sind.

Die prioritätsältere, nachveröffentlichte WO 2014/075808 A2 betrifft einen Prothesenschaft mit einem Schaftkörper und einem flexiblen Inlay, das bei angelegtem Prothesenschaft zwischen dem Schaftkörper und dem Stumpf angeordnet ist. Das Inlay ist mit dem Schaftkörper an dessen proximalen Bereich fest verbunden.

Der Erfindung liegt von daher die Aufgabe zugrunde, einen Prothesenschaft der eingangs genannten Art zu schaffen, mit dem insbesondere das Anziehen einer Prothese erleichtert wird.

Diese Aufgabe wird erfindungsgemäß durch einen Prothesenschaft mit den Merkmalen des Anspruchs 1 gelöst. Vorteilhafte Ausführungsformen der Erfindung sind in den weiteren Ansprüchen beschrieben.

Beim erfindungsgemäßen Prothesenschaft umfasst die Einführhilfseinrichtung ein Einführinlay, das in seinem proximalen Randbereich mit einer Halteeinrichtung verbunden ist, welche an dem Schaftkörper fixiert ist. Ferner weist das Einführinlay einen Einführstrumpf auf, der zwischen einer äußeren Position, in der sich das Einführinlay zumindest teilweise, vorzugsweise überwiegend, außerhalb des Schaftkörpers befindet, und einer inneren Position bewegbar ist, in welcher der Einführstrumpf den Schaftkörper auskleidet.

Das Einführinlay weist elastische, druckstabile Aufspannelemente auf, die sich entlang des Einführstumpfes erstrecken, an diesem fixiert sind und zusammen mit dem Einführstrumpf bewegbar sind. Diese Aufspannelemente haben die Aufgabe, beim Ausziehen des Prothesenschaftes einen Druck, der beispielsweise durch eingeleitete Druckluft auf das distale Ende des Einführinlays ausgeübt wird, in proximale Richtung weiterzuleiten. Dadurch wird es möglich, einen Amputationsstumpf einfach aus dem Einführinlay zu entfernen, ohne dass es zu Quetschungen von Weichteilen kommt. Zudem werden Faltenwürfe beim Einführinlay vermieden oder zumindest stark verringert.

Die Aufspannelemente müssen daher so druckstabil sein, dass sie den beispielsweise an ihrem distalen Ende aufgebrachten Druck zu ihrem proximalen Ende hin weiterleiten können. Dennoch benötigen sie eine gewisse Biegefähigkeit, um der sich beim Ausziehen des Prothesenschaftes ergebenden Veränderung der Kontur des Einführinlays und insbesondere des Einführstrumpfes anpassen und dieser folgen zu können. Dabei ist es von Vorteil, wenn diese beim Ausziehen auftretende Verbiegung oder Durchbiegung der einzelnen Aufspannelemente ausgeführt werden kann, ohne dass es zu einem Knicken oder Verknicken der Aufspannelemente kommt. Sie werden daher im Folgenden auch als knickstabil beschrieben.

Vorzugsweise ist die Halteeinrichtung relativ zu dem proximalen Schaftrand des Schaftkörpers beweglich. Insbesondere in Kombination mit elastischen, druckstabilen Aufspannelementen führt dies dazu, dass beim Ausziehen des Prothesenschaftes und beim Entfernen des Amputationsstumpfes aus dem Einführstrumpf, der Einführstrumpf und dabei auch das Einführinlay aufgeweitet wird, so dass der Amputationsstumpf einfach entfernt und zu einem späteren Zeitpunkt wieder einfach in das Einführinlay eingebracht werden kann.

Vorzugsweise ist die Halteeinrichtung an der Außenwand des Schaftkörpers fixiert. Besonders vorzugsweise ist sie dabei mit ihrem distalen Ende fixiert.

Mit Hilfe des erfindungsgemäßen Einführinlays wird das Einführen des Amputationsstumpfs in den Schaftkörper bedeutend erleichtert. Es ist nicht mehr erforderlich, vor dem Einführvorgang einen Liner auf den Amputationsstumpf aufzuziehen. Vor dem Einführen des Amputationsstumpfs in den Schaftkörper erstreckt sich das Einführinlay proximal über den proximalen Schaftrand hinaus, wobei der proximale Abschnitt des Einführinlays durch die Aufspannelemente derart weiter radial nach außen aufgespannt und geweitet wird, dass der Amputationsstumpf ohne weiteres auf das Einführinlay aufgesetzt werden kann. Beim weiteren Einführen des Amputationsstumpfes in den Schaftkörper gleitet das Einführinlay längs des Schaftkörpers in distaler Richtung, bis der Amputationsstumpf am Boden des Prothesenschafts aufliegt. Bei diesem Einführvorgang wird der Amputationsstumpf entsprechend dem reduzierten Durchmesser des Schaftkörpers in der gewünschten Weise komprimiert. Der Einführvorgang kann dabei gegebenenfalls mittels einer Seilwinde unterstützt werden, mit welcher das Einführinlay oder ein weiteres Schaftinlay, das sich zwischen dem Einführinlay und dem Schaftkörper befindet, in distaler Richtung gezogen wird. Beim Einführvorgang sorgen die Aufspannelemente dafür, dass der elastische, dehnbare Einführstrumpf faltenfrei in den Schaftkörper gelangt und diesen letztendlich auskleidet.

Besonders vorteilhaft ist das erfindungsgemäße Einführinlay in Kombination mit einem ausstülpbaren Schaftinlay, das zwischen dem sich in seiner inneren Position befindenden Einführinlay und dem Schaftköper angeordnet ist und zusammen mit dem Schaftkörper oder allein einen fluiddichten Fluiddruckraum begrenzt, so dass das Schaftinlay durch Erzeugen eines Überdrucks im Fluiddruckraum in proximaler Richtung aufstülpbar ist. Zweckmäßigerweise erfolgt der Ausstülpvorgang mittels Druckluft. Der Amputationsstumpf wird dabei durch das Fluid aus dem Schaftkörper hinausgedrückt. Hierdurch wird das Abziehen des Amputationsstumpfs vom Prothesenschaft bedeutend erleichtert. Dieses Schaftinlay muss, damit der Ausstülpvorgang gleichmäßig und symmetrisch erfolgt, am Schaftkörper in einer Schafteintrittsebene befestigt sein, die senkrecht zur Stumpfeinführrichtung angeordnet ist. Der starre Schaftkörper erstreckt sich in der Regel jedoch über diese Schafteintrittsebene hinaus und ist jenseits (proximal) der Schafteintrittsebene nicht mehr durch das Schaftinlay ausgekleidet. Das Einführinlay, das sich auch über den proximalen Randbereich des Schaftkörpers erstreckt, verringert ganz wesentlich die Reibung im proximalen Randbereich des Schaftkörpers beim Einführen des Amputationsstumpfs. Zugschmerzen auf der Haut und eine Stauchung der Weichteile oberhalb des proximalen Randbereichs des Schaftkörpers können wesentlich verringert werden.

Ein weiterer Vorteil des erfindungsgemäßen Einführinlays liegt darin, dass zu Beginn des Einführvorgangs immer eine Aufsetzplattform für den Amputationsstumpf geschaffen wird, die senkrecht zur Einführrichtung liegt. Ist die Einführrichtung vertikal, liegt diese Aufsetzplattform somit in einer horizontalen Ebene. Dies ist auch dann der Fall, wenn der proximale Schaftrand in üblicher Weise schief und wellig verläuft. Die relativ zum proximalen Schaftrand bewegliche Halteeinrichtung stellt dabei sicher, dass das Einführinlay über den proximalen Schaftrand hinaus in der gewünschten Weise insbesondere regenschirm- oder pilzartig aufgespannt wird, wenn das Einführinlay in seine äußere Position gebracht wird.

Vorteilhafterweise ist die Halteeinrichtung in Höhe einer Schafteintrittsebene, die senkrecht zur Stumpfeinführrichtung liegt, am Schaftkörper fixiert. Dies stellt sicher, dass aufgrund der unterschiedlichen Verbindungslängen der Halteeinrichtung, die durch den schiefen bzw. welligen Randverlauf des Schaftkörpers bedingt sind und in der Regel medial kürzer als lateral sind, das Einführinlay so umgestülpt wird, dass medial und lateral sowie anterior und posterior derartige, gegebenenfalls auch unterschiedlich große Auswölbungen des Einführinlays stattfinden können, dass das Einführinlay faltenfrei aufgespannt wird.

Zweckmäßigerweise besteht die Halteeinrichtung aus mehreren um den Umfang des Schaftkörpers herum angeordneten Haltebänder oder -seilen. Diese Haltebänder oder -seile können unelastisch oder elastisch ausgebildet sein. Alternativ ist es auch möglich, dass die Halteinrichtung aus einem schlauchartigen Halteorgan besteht, das den Schaftkörper umgibt.

Die Haltebänder oder -seile sind vorteilhafterweise jeweils an einem Aufspannelement befestigt. Alternativ können sie jedoch auch am proximalen Rand des Einführstrumpfs befestigt sein.

Vorteilhafterweise bestehen die Aufspannelemente aus länglichen, biegsamen Führungsstäben oder -leisten, die mit dem Einführstrumpf fest verbunden sind, beispielsweise verklebt oder direkt aufgespritzt sind, oder in Führungslaschen oder -taschen des Einführstrumpfs eingesetzt sind.

Vorteilhafterweise ist eine Mehrzahl von Aufspannelementen über den Umfang des Einführstrumpfs herum mit einem zumindest annähernd gleichen gegenseitigen Abstand angeordnet. Anzahl und Anordnung der Aufspannelemente können jedoch variiert und an die geometrischen Gegebenheiten und an das Material des Einführstrumpfs angepasst werden.

Vorteilhafterweise erstrecken sich die Aufspannelemente vom Boden des Einführstrumpfs bis zu dessen proximalen Randbereich. Hierdurch wird auf besonders wirkungsvolle Weise ein Stauchen des Einführstrumpfs beim Überführen in dessen äußere Position verhindert.

Vorteilhafterweise ist das Einführinlay in seiner äußeren Position zumindest überwiegend umgestülpt, wobei sich der Boden des Einführstrumpfes außerhalb des Schaftköpers oder auf gleicher Höhe wie der am weitesten proximal vorstehende Schaftrand befindet. Hierdurch wird ein besonders einfaches Ansetzen und Einführen des Amputationsstumpfes in den Prothesenschaft ermöglicht.

Gemäß einer vorteilhaften Ausführungsform ist zwischen dem sich in seiner inneren Position befindenden Einführinlay und dem Schaftkörper ein flexibles Schaftinlay angeordnet, das zusammen mit dem Schaftkörper oder allein einen fluiddichten Fluiddruckraum begrenzt, durch Erzeugen eines Überdrucks im Fluiddruckraum in proximaler Richtung ausstülpbar ist und einen relativ zum Schaftkörper verschiebbaren Boden aufweist, wobei das Einführinlay im Bereich des Bodens mit dem Schaftinlay verbunden ist. Bei dieser Ausführungsform kann das Einführinlay durch das Schaftinlay mittels Fluid, insbesondere mittels Druckluft, proximal nach außen gedrückt und pilzartig in einen größeren Umfang außerhalb des Schaftkörpers umgestülpt werden. Die Aufspannelemente biegen sich dabei aufgrund der Befestigung an der Halteeinrichtung federartig radial nach außen und dehnen dabei das Einführinlay im Umfang auf, wobei dieses regenschirm- oder pilzartig umgestülpt wird.

Vorteilhafterweise bestehen die Aufspannelemente aus Kunststoff wie beispielsweise PU, PE, PP, einem faserverstärkten Kunststoff oder Silikon, Co-Polymeren oder ähnlichen Stoffen, oder aus einem Federstahl.

Vorteilhafterweise ist der Boden des Schaftinlays als formstabile Kappe ausgebildet. Eine derartige Kappe bewirkt, dass der Umstülpvorgang in ordnungsgemäßer und gleichmäßiger Weise längs des Prothesenschafts erfolgt. Weiterhin wird durch die Kappe bei ausgestülptem Inlay eine schalenartige Vertiefung zum Einsetzen des Amputationsstumpfes geschaffen, die eine vollflächige Kontaktierung mit der distalen Stirnseite des Amputationsstumpfes ermöglicht.

Vorteilhafterweise ist das Schaftinlay mit dem Schaftkörper in einer senkrecht zur Stumpfeinführrichtung liegenden Schafteinsatzebene luftdicht verbunden. Bei dieser Ausführungsform ist zwischen dem sich in der inneren Position befindenden Einführinlay und dem Schaftkörper eine einzige Lage des Schaftinlays angeordnet, das zusammen mit dem Schaftkörper den Fluiddruckraum begrenzt. Alternativ hierzu ist es jedoch auch möglich, das Schaftinlay als fluiddichte Blase auszubilden, deren beide Hälften ineinander gestülpt und im Zwischenraum zwischen Schaftkörper und Einführinlay angeordnet sind. Zum Ausstülpen des Schaftinlays wird dann Fluid in das Innere der Inlayblase eingeleitet. Bei dieser Ausführungsform ist es nicht erforderlich, das Schaftinlay gegenüber dem Schaftkörper abzudichten.

Vorteilhafterweise verfügt der Prothesenschaft über wenigstens ein Zugelement, durch das ein distales Ende des Einführinlays in Richtung auf ein distales Ende des Schaftkörpers bewegbar ist. Dieses Zugelement kann beispielsweise das bereits genannte Zugseil sein, das über eine Seilwinde bewegbar ist. Es sind jedoch auch Ausgestaltungen denkbar, bei denen beispielsweise ein Zugseil von Hand betätigt wird, um das Einführinlay in den Schaftkörper einbringen zu können. Eine besonders komfortable Möglichkeit besteht darin, am distalen Ende des Schaftkörpers beispielsweise eine Umlenkrolle oder eine sonstige Umlenkung vorzusehen, durch die das Zugelement umgelenkt wird. Damit wird es für den Träger der Prothese und des Prothesenschaftes möglich, am Zugelement nach oben, also in proximaler Richtung, zu ziehen, um das Einführinlay in den Schaftkörper einbringen zu können. Als Zugelement kann insbesondere ein Zugseil, eine Zugkette oder ein Zugband vorhanden sein, wobei eine Zugkette und ein Zugband den Vorteil aufweisen, dass sie nicht aufgewickelt werden müssen. Damit ist eine platzsparendere Ausgestaltung möglich. Ein Zugband hat zudem den Vorteil, dass es durch eine entsprechende Fördereinrichtung förderbar ist. Natürlich sind auch mehrere Zugelemente, gegebenenfalls unterschiedlicher oder identischer Ausgestaltung denkbar.

Die Erfindung wird nachfolgend an Hand der Zeichnungen beispielhaft näher erläutert. Es zeigen:
- Figur 1:: eine räumliche Darstellung des erfindungsgemäßen Prothesenschafts,
- Figur 2:: einen Vertikalschnitt durch den Prothesenschaft von Figur 1, wobei ein Detail vergrößert dargestellt ist,
- Figur 3:: einen Vertikalschnitt des Prothesenschaftes, wobei sich die Inlays in einem ausgestülpten Zustand befinden, und
- Figuren 4a-4d:: den Prothesenschaft beim Einführen eines Amputationsstumpfes, wobei vier unterschiedliche Einführpositionen dargestellt sind.

Die Figuren zeigen einen Prothesenschaft 1 für eine Beinprothese.

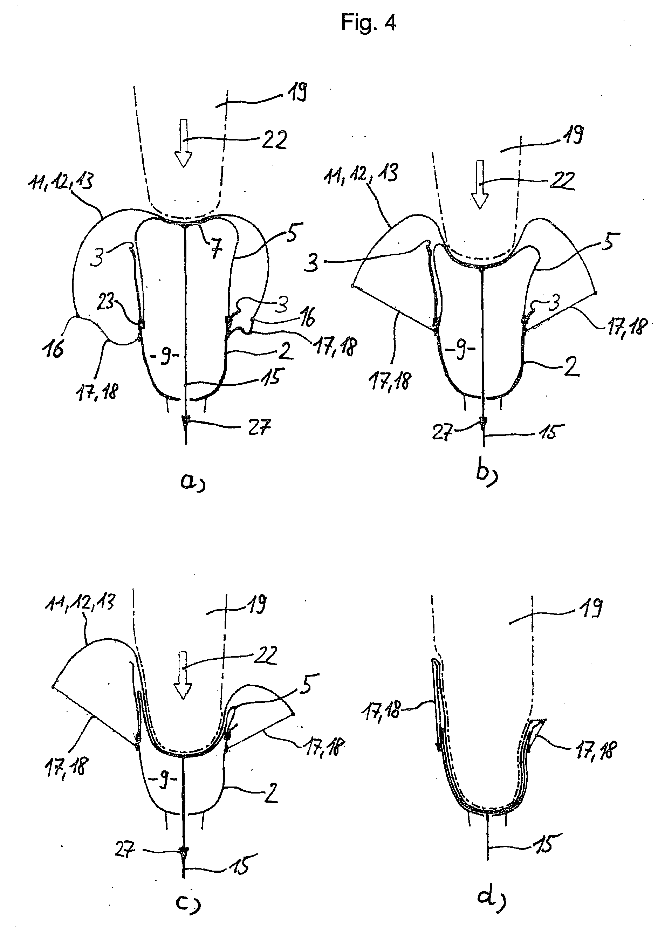

Der Prothesenschaft 1 umfasst einen eimer- oder napfförmigen Schaftkörper 2 aus starrem Material, insbesondere aus Kunststoff. Am proximalen Ende weist der Schaftkörper 2 eine proximale Einführöffnung 3 für den lediglich in Figur 4 andeutungsweise dargestellten Amputationsstumpf 19 auf. An seinem distalen Ende ist der Schaftkörper 2 zumindest weitgehend geschlossen. Der Schaftkörper 2 umschließt einen Innenraum 4.

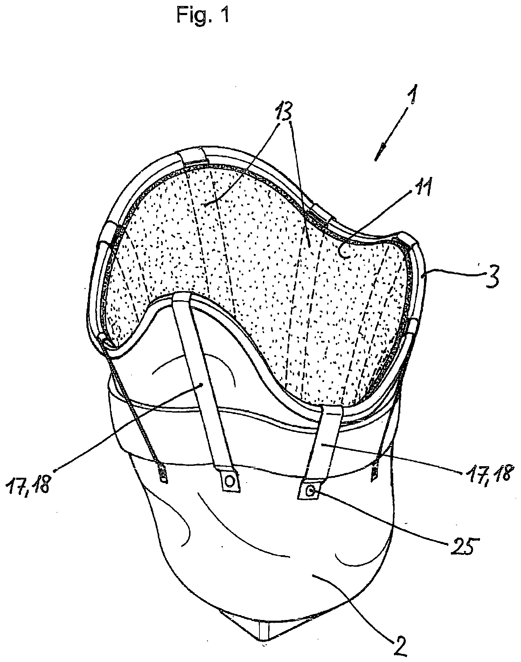

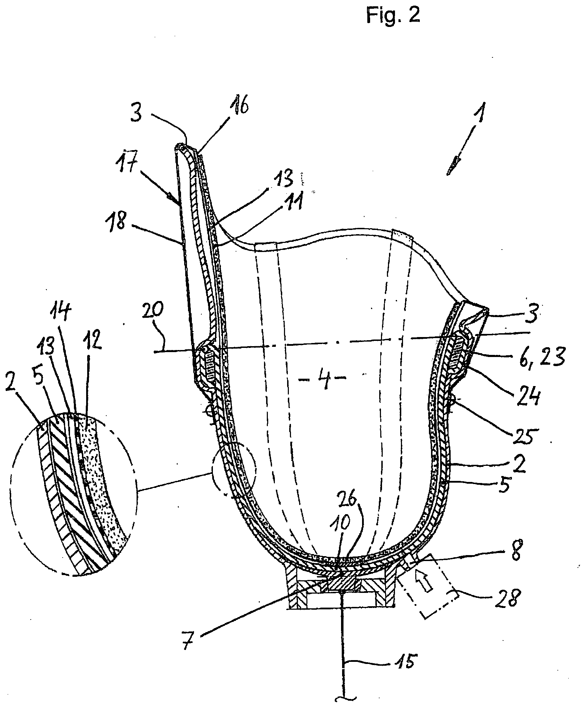

Der Prothesenschaft 1 umfasst ferner ein flexibles Schaftinlay 5 aus einem elastischen Material wie Silikon, Polyurethan, Co-Polymer oder ähnlichen Materialien. In der in den Figuren 1 und 2 gezeigten Lage des Schaftinlays 5, die derjenigen entspricht, die bei eingesetztem Amputationsstumpf 19 eingenommen wird, überdeckt das Schaftinlay 5 den Schaftkörper 2 in dem gesamten Bereich zwischen einer Schafteintrittsebene 20 und dem Boden des Schaftkörpers 2. Das Schaftinlay 5 dient damit einerseits als Polsterung und andererseits als elastische Zwischenschicht, die eine vollflächige, möglichst druckspitzenfreie Kraftübertragung zwischen Amputationsstumpf 19 und Schaftkörper 2 ermöglicht.

Als Schafteintrittsebene 20 wird hier diejenige Ebene bezeichnet, die senkrecht zur Einführrichtung 22 (Figur 4) des Amputationsstumpfs 19 liegt und im dargestellten Ausführungsbeispiel horizontal angeordnet ist.

Das Schaftinlay 5 ist lediglich in seinem proximalen Endbereich mit einer ringförmig umlaufenden Verbindung 6 auf der Innenseite des Schaftkörpers 2 in Höhe der Schafteintrittsebene 20 befestigt. Diese Verbindung 6 wird im dargestellten Ausführungsbeispiel durch einen in Umfangsrichtung des Schaftkörpers 2 umlaufenden Klemmring 23 realisiert, der in eine nach außen gewölbte Sicke 24 des Schaftkörpers 2 eingelegt ist und eine radiale Spannkraft nach außen erzeugt. Das proximale Ende des Schaftinlays 5 ist innerhalb der Sicke 24 durch den Klemmring 23 festgeklemmt und luftdicht gegenüber dem Schaftkörper 2 abgedichtet.

Anstelle einer derartigen Verbindung 6 sind auch andere Verbindungen, beispielsweise Klebeverbindungen, möglich.

Das Schaftinlay 5 besteht aus einem fluiddichten elastischen, gut dehnbaren Material. Der Zwischenraum zwischen Schaftkörper 2 und Schaftinlay 5 ist daher fluiddicht abgedichtet. Weiterhin liegt das Schaftinlay 5 in den von der Verbindung 6 entfernten Bereichen lediglich lose an der inneren Oberfläche des Schaftkörpers 2 an.

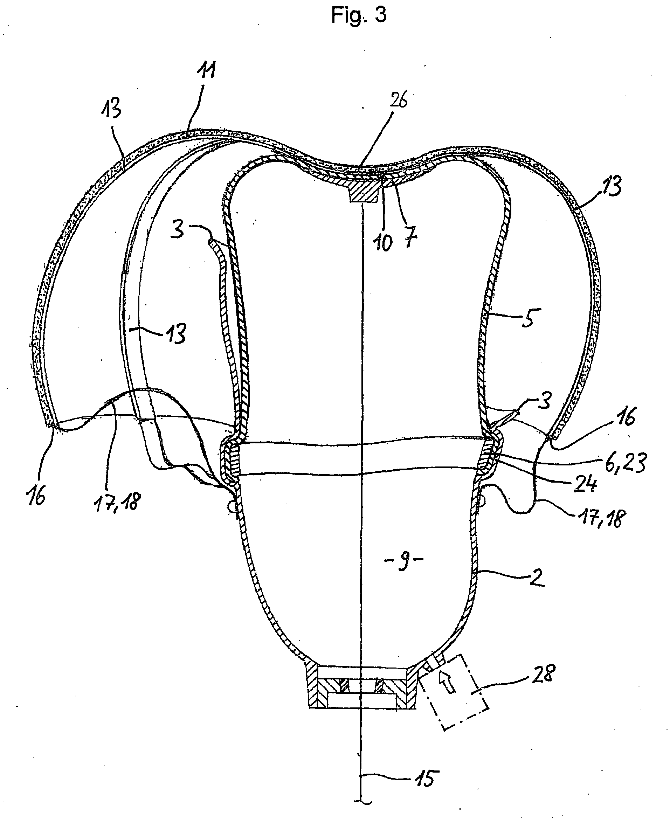

Im distalen Endbereich des Prothesenschaftes 1 befindet sich weiterhin eine Überdruckerzeugungseinrichtung 28, die am Schaftkörper 2 befestigt oder mit diesem in Fluidverbindung bringbar ist. Diese Überdruckerzeugungseinrichtung 28 ist über einen Fluidkanal 8 mit dem Zwischenraum zwischen dem Schaftkörper 2 und dem Schaftinlay 5 verbunden, um bei Bedarf Fluid in diesen Zwischenraum einzupumpen und darin einen Überdruck zu erzeugen. Dieser Zwischenraum stellt damit einen Fluiddruckraum dar, der in den Figuren 3 und 4 mit dem Bezugszeichen 9 bezeichnet ist. Zweckmäßigerweise handelt es sich bei dem Fluid um Luft und bei der Überdruckerzeugungseinrichtung 28 um einen elektrisch betriebenen Kompressor, mit dem Druckluft in den Fluiddruckraum 9 eingepumpt werden kann.

Durch das Einpumpen von Fluid in den Fluiddruckraum 9 wird auf einen Boden 10 des Schaftinlays 5, der in Figur 2 im Bereich des Bodens des Schaftkörpers 2 angeordnet und außenseitig mit einer formstabilen Kappe 7 verstärkt ist, eine Verschiebekraft in proximaler Richtung ausgeübt. Dies bewirkt ein Verschieben des Bodens 10 in proximaler Richtung relativ zum Schaftkörper 2. Bei dieser Verschiebung wird, wie aus den Figuren 3 und 4 ersichtlich, das Schaftinlay 5 umgestülpt, wobei sich der Boden 10 innerhalb der Seitenwand des Schaftkörpers 2 in proximaler Richtung mindestens bis zu dem am weitesten proximal liegenden Schaftrand 3 und im gezeigten Ausführungsbeispiel sogar darüber hinaus bewegt.

Um zu verhindern, dass sich das Schaftinlay 5 innerhalb des Schaftkörpers 2 um die Längsachse dreht, können einerseits am Schaftkörper 2 und andererseits am Schaftinlay 5 rotationshemmende Elemente vorgesehen sein, die formschlüssig zusammenwirkende Stege und zwischen den Stegen vorgesehene Nuten umfassen, die in Längsrichtung des Prothesenschafts 1 verlaufen. Zweckmäßigerweise sind diese rotationshemmenden Elemente im mittleren und/oder distalen Drittel des Prothesenschafts 1 angeordnet, da das Schaftinlay 5 am proximalen Ende bereits durch die fluiddichte Verbindung 6 daran gehindert wird, sich relativ zum Schaftkörper 2 zu verdrehen. Die am Schaftinlay 5 vorgesehenen rotationshemmenden Elemente sind darüber hinaus flexibel ausgebildet, um das Umstülpen des Schaftinlays 5 auch im Bereich der rotationshemmenden Elemente zu ermöglichen.

Alternativ oder zusätzlich ist es auch möglich, entsprechende rotationshemmende Elemente zwischen dem Boden 10 des Schaftinlays 5 bzw. der Kappe 7 und dem benachbarten Boden des Schaftkörpers 2 vorzusehen.

Um den Prothesenschaft 1 auf möglichst einfache Weise mit dem Amputationsstumpf 19 verbinden zu können, ist weiterhin eine Seilzugvorrichtung vorgesehen, die eine nicht dargestellte Seilwinde und ein von dieser betätigbares Zugseil 15 umfasst. Die Seilwinde ist in einer nicht näher dargestellten Weise am distalen Ende des Schaftkörpers 2 befestigt. Weiterhin ist das Zugseil 15 durch eine nicht dargestellte Zugseildurchführung hindurchgeführt, die gegenüber dem Fluiddruckraum 9 abgedichtet ist und verhindert, dass beim Erzeugen eines Überdrucks im Fluiddruckraum 9 das Fluid in unerwünschter Weise durch die Zugseildurchführung abströmen kann. Diese Abdichtung kann beispielsweise durch nicht dargestellte flexible Dichtlippen erfolgen, die am Zugseil 15 anliegen. Weiterhin kann es bereits genügen, den Spalt zwischen Zugseil 15 und Zugseildurchführung so eng auszuführen, dass die Menge des hindurchtretenden Fluids vernachlässigbar ist.

Das Zugseil 15 ist an seinem proximalen Ende mittig mit dem Boden 10 des Schaftinlays 5 beziehungsweise mit der formstabilen Kappe 7 verbunden. Aufgrund dieser Anordnung kann der Boden 10, ausgehend von der in den Figuren 3 und 4a gezeigten expandierten Stellung des Schaftinlays 5, mittels der Seilwinde in distaler Richtung gezogen und damit in den Schaftkörper 2 eingezogen werden. Das Schaftinlay 5 wird dabei zurückgestülpt, bis es die in den Figuren 1 und 2 dargestellte Lage einnimmt.

Wie aus den Figuren 1 bis 3 ersichtlich, erstreckt sich der Schaftkörper 2 proximal über die Schafteintrittsebene 20 hinaus, wobei der Abstand zwischen dem proximalen Schaftrand 3 und der Schafteintrittsebene 20 variiert. Weiterhin verläuft der proximale Schaftrand 3 des Schaftkörpers 2 wellig. Der Randverlauf wird in der Regel individuell an den Patienten angepasst. Bei der dargestellten Beinprothese wird zum Beispiel lateral der Trochanter major komplett mit gefasst, während im Perineum ein tiefer Ausschnitt genug Platz für die Adduktorensehne und Ramus sicherstellen muss. Andererseits wäre es unvorteilhaft, das Schaftinlay 5 längs und in unmittelbarer Nachbarschaft des unregelmäßigen proximalen Schaftrandes 3 zu befestigen, da dann kein gleichmäßiges, symmetrisches und faltenfreies Ausstülpen des Schaftinlays 5 längs der Stumpfeinführrichtung möglich wäre, sondern das Schaftinlay 5 unregelmäßig und schräg ausgestülpt werden würde. Die Befestigung des Schaftinlays 5 mit distalem Abstand zum proximalen Schaftrand 3, wie in den Figuren dargestellt, hat jedoch zur Folge, dass der Bereich des Schaftkörpers 2 zwischen der Schafteintrittsebene 20 und dem proximalen Schaftrand 3 nicht mehr vom Schaftinlay 5 bedeckt wird. Die Haut des Amputationsstumpfs 19 würde daher an diesem nicht überdeckten proximalen Schaftkörperbereich anliegen, was aufgrund der hohen Reibung zu Zugschmerzen auf der Haut und zu einer Stauchung der Weichteile oberhalb des proximalen Randes 3 führen kann. Weiterhin könnte die erhöhte Reibung zwischen dem Amputationsstumpf und dem oberhalb der Schafteintrittsebene 20 liegenden Schaftkörperbereich dazu führen, dass ein vollständiges Anziehen des Prothesenschafts 1 nicht möglich ist.

Um diesem Problem abzuhelfen, weist der Prothesenschaft 1 eine zusätzliche Einführhilfseinrichtung auf, die das Einführen des Amputationsstumpfs 19 in den Schaftkörper 2 erleichtert. Die Einführhilfseinrichtung umfasst ein zusätzliches Inlay, das hier als Einführinlay 11 bezeichnet wird. In seiner inneren Position, die in den Figuren 1 und 2 dargestellt ist, kleidet das Einführinlay 11 den Schaftkörper 2 vollständig aus, wobei es sich vom distalen Ende des Schaftkörpers 2 bis zum proximalen Schaftrand 3 oder zumindest bis in die Nähe des proximalen Schaftrands 3 erstreckt. Das Einführinlay 11 überdeckt somit sowohl das Schaftinlay 5 als auch denjenigen Schaftkörperbereich, der proximal zur Schafteintrittsebene 20 liegt.

Das Einführinlay 11 umfasst einen Einführstrumpf 12 und elastische, biegsame, jedoch knickstabile Aufspannelemente 13, die auf der Außenseite, das heißt der dem Schaftkörper 2 zugewandten Seite, des Einführstrumpfs 12 angeordnet und mit diesem fest verbunden sind. Diese Verbindung kann beispielsweise durch eine Klebeschicht 14 erfolgen (siehe Detailzeichnung von Figur 2).

Der Einführstrumpf 12 kann beispielsweise aus Silikon, PU oder Co-Polymer oder ähnlichen Kunststoffen hergestellt sein. Zweckmäßigerweise wird ein Material verwendet, das zumindest auf der dem Schaftkörper 2 zugewandten Seite gute Gleiteigenschaften hat. Es ist auch ohne weiteres möglich, den Einführstrumpf 12 auf dieser Seite mit einem speziellen Gleitmaterial zu beschichten.

Bei den Aufspannelementen 13 handelt es sich um längliche, leicht biegsame, jedoch knickstabile Führungsstäbe oder -leisten, die sich vom Boden 26 des Einführinlays 11 bzw. Einführstrumpfs 12 (Figur 2) in Längsrichtung des Einführstrumpfs 12 bis zu dessen proximalem Randbereich 16 erstrecken. Da der proximale Randverlauf des Einführstrumpfs 12 an den unterschiedlich hohen, welligen proximalen Schaftrand 3 angepasst ist und sich bis in die gleiche Höhe erstreckt, ist die Länge der beidseits zur Längsmittelachse des Prothesenschafts 1 angeordneten Aufspannelemente 13 unterschiedlich, je nachdem, ob das entsprechende Aufspannelement 13 überwiegend medial, lateral, anterior oder posterior angeordnet ist. Wie weiterhin aus den Figuren 1 bis 3 ersichtlich, sind eine Mehrzahl von Aufspannelementen 13 über den Umfang des Einführstrumpfs 12 angeordnet, wobei im gezeigten Ausführungsbeispiel acht Aufspannelemente 13 vorgesehen sind. Diese Zahl kann jedoch in weitem Umfang, beispielsweise zwischen drei und dreißig, variieren. Zweckmäßigerweise sind die Aufspannelemente 13 zumindest einigermaßen regelmäßig um den Umfang des Einführstrumpfs 12 herum angeordnet, so dass sie zueinander in etwa den gleichen Abstand haben.

Das gesamte Einführinlay 11 liegt in der inneren Position lose innerhalb des Schaftkörpers 2 und ist lediglich in seinem proximalen Randbereich 16 an einer beweglichen Halteeinrichtung 17 sowie im Bereich seines Bodens 26 am Boden 10 des Schaftinlays 11 befestigt.

Um zu verhindern, dass sich das Einführinlay 11 bei eingeführtem Amputationsstumpf 19 in unerwünschter Weise gegenüber dem Schaftinlay 5 verschiebt oder verdreht, ist das Schaftinlay 5 auf seiner dem Einführinlay 11 zugewandten Seite zweckmäßigerweise mit einer reibungserhöhenden Schicht, insbesondere einer Silikonschicht, versehen.

Die Halteeinrichtung 17 umfasst im dargestellten Ausführungsbeispiel mehrere um den Umfang des Schaftkörpers 2 herum angeordnete Haltebänder 18, die in der in den Figuren 1 und 2 dargestellten inneren Position des Einführinlays 11 an ihrem proximalen Ende geringfügig um den proximalen Schaftrand 3 herumgeführt und mit dem proximalen Randbereich 16 des Einführinlays 11 verbunden sind, während sie im Übrigen außerhalb des Schaftkörpers 2 nach Distal geführt und an ihrem distalen Ende in einer Befestigungsebene, die parallel zur Schafteintrittsebene 20 liegt, an der Außenseite des Schaftkörpers 2 befestigt sind. Im gezeigten Ausführungsbeispiel erfolgt die Befestigung der distalen Enden der Haltebänder 18 mittels Nieten 25. Weiterhin ist jedes Aufspannelement 13 an einem Halteband 18 befestigt. Die Länge der Haltebänder 18 ist so bemessen, dass sie in der inneren Position des Einführinlays 11 gespannt oder zumindest nahezu gespannt sind.

Die Halteeinrichtung 17 bildet damit eine Ankereinrichtung für den proximalen Endbereich des Einführinlays 11, wobei die Ankereinrichtung eine gewisse Bewegung dieses proximalen Endbereichs über den proximalen Schaftrand 3 hinaus radial nach außen ermöglicht, diese Bewegung jedoch auf ein Maß begrenzt, das durch die Länge der Haltebänder 18 bestimmt ist.

Der Boden 26 des Einführinlays 11, das heißt derjenige Endbereich, der in der inneren Position distal angeordnet ist, ist am Boden 10 des Schaftinlays 5 befestigt. Diese Befestigung kann beispielsweise mittels einer Klebung erfolgen. Die Befestigung ist jedoch ausschließlich auf diesen distalen Endbereich 10 beschränkt, während die übrigen Bereiche des Einführinlays 11 keine weitere feste Verbindung zum Schaftinlay 5 haben.

Wird das Schaftinlay 5 von der in Figur 2 gezeigten inneren Position in die in Figur 3 dargestellte expandierte Position ausgestülpt, bewegt sich der Randbereich 16 des Einführinlays 11 über den proximalen Schaftrand 3 hinaus nach oben und radial nach außen und wird anschließend mit radialem Abstand zum Schaftkörper 2 durch die Haltebänder 18 wieder etwas nach unten, das heißt distal, gezogen. Da die Aufspannelemente 13 zwar biegsam, jedoch knickstabil sind, kommt es zu einem regenschirm- oder pilzartigen Aufspannen des Einführinlays 11. Die Aufspannelemente 13 wirken damit als Führungsstreben für den Einführstrumpf 12. Der aus einem elastischen, dehnbaren Material bestehende Einführstrumpf 12 wird dabei durch die Federkraft der Aufspannelemente 13 faltenfrei aufgespannt, bis sich der Einführstrumpf 12 nicht mehr weiter dehnt. Wird das Schaftinlay 5 durch das eingeleitete Fluid weiter bis in seine maximale Endstellung ausgestülpt, die in Figur 3 dargestellt ist, wird der proximale Randbereich 16 des Einführinlays 11 mittels der Aufspannelemente 13 weiter in distaler Richtung vorgeschoben, wobei die pilzartige Form des Einführinlays 11 durch die Dehnung des Einführstrumpfs 12 bestimmt wird. In der Endstellung, die in Figur 3 gezeigt ist, können daher die Haltebänder 18 entspannt sein.

Aus Figur 3 ist erkennbar, dass dabei der Boden 26 des Einführinlays 11 zusammen mit dem Boden 10 des Schaftinlays 5 gleichmäßig senkrecht zur Stumpfeinführrichtung, das heißt in Figur 3 horizontal, angeordnet ist.

Im Folgenden wird an Hand von Figur 4 das Einführen des Amputationsstumpfes 19 in den Prothesenschaft 1 näher erläutert.

Figur 4a zeigt die expandierte Stellung des Schaftinlays 5, wobei diese Stellung derjenigen von Figur 3 entspricht. Es ist ersichtlich, dass der Amputationsstumpf 19 zunächst von oben her in Einführrichtung 22 auf den Boden 26 des Einführinlays 11 und damit auch auf den Boden 10 des Schaftinlays 5 aufgesetzt wird. Gleichzeitig wird mittels einer nicht dargestellten Seilwinde eine Zugkraft auf das Zugseil 15 aufgebracht, wie durch den Pfeil 27 angedeutet.

Mittels des Zugseils 15 und durch eine gewisse, jedoch relative geringe Druckkraft des Amputationsstumpfs 19 werden anschließend Schaftinlay 5 und Einführinlay 11 zusammen in den Schaftkörper 2 eingeführt, wie aus Figur 4b ersichtlich. Die Aufspannelemente 13 des Einführinlays 11 biegen sich dabei oberhalb des proximalen Schaftrands 3, ohne abzuknicken. Der Randbereich 16 des Einführinlays 11 wird dabei mittels der mittlerweile gespannten Haltebänder 18 kreisförmig um die distalen Befestigungspunkte der Haltebänder 18 herumgeführt.

Wird der Amputationsstumpf 19, wie aus Figur 4c ersichtlich, weiter in den Prothesenschaft 1 eingeführt, beginnt die radiale Kompression des Amputationsstumpfes 19, da dieser einen geringeren Durchmesser als der Amputationsstumpf 19 hat. Mittels des Zugseils 15 wird der Boden 10 des Schaftinlays 5 und damit auch der Boden 26 des Einführinlays 11 zunehmend in distaler Richtung eingezogen, wobei das Einführinlay 11 während des gesamten Einführvorgangs eine leicht längs des Schaftkörpers 2 gleitende Zwischenschicht zwischen Amputationsstumpf 19 und Schaftkörper 2 bildet.

In der in Figur 4d gezeigten Stellung befindet sich das Einführinlay 11 in seiner inneren Position, in welcher es den Schaftkörper 2 vollständig auskleidet.

Im Rahmen der Erfindung sind eine Vielzahl von Variationen möglich. Beispielsweise kann das erfindungsgemäße Einführinlay 11 auch ohne zusätzlichem Schaftinlay 5 verwendet werden. Das Zugseil 15 ist in diesem Fall am Boden 26 des Einführinlays 11 befestigt und kann auch verwendet werden, um das Einführinlay 11 am Boden des Schaftkörpers 2 zu fixieren. Anstelle einer Seilwinde kann das Zugseil 15 auch manuell betätigt werden.

## Patentansprüche

1. Prothesenschaft (1) mit einem Schaftkörper (2) zur Aufnahme eines Amputationsstumpfs (19) und einer Einführhilfseinrichtung, die das Einführen des Amputationsstumpfs (19) in den Schaftkörper (2) erleichtert, wobei die Einführhilfseinrichtung ein Einführinlay (11) umfasst, das in seinem proximalen Randbereich (16) mit einer Halteeinrichtung (17) verbunden ist, welche an dem Schaftkörper (2) fixiert ist, wobei das Einführinlay (11) einen Einführstrumpf (12) aufweist, der zwischen einer äußeren Position, in der sich das Einführinlay (11) zumindest teilweise, vorzugsweise zumindest überwiegend, außerhalb des Schaftkörpers (2) befindet, und einer inneren Position bewegbar ist, in welcher der Einführstrumpf (12) den Schaftkörper (2) auskleidet, **dadurch gekennzeichnet, dass** das Einführinlay (11) elastische, druckstabile Aufspannelemente (13) aufweist, die sich entlang des Einführstrumpfes (12) erstrecken, an diesem fixiert sind und zusammen mit dem Einführstumpf (12) bewegbar sind.

2. Prothesenschaft nach Anspruch 1, **dadurch gekennzeichnet, dass** die Halteeinrichtung (17) relativ zu dem proximalen Schaftrand (3) des Schaftkörpers (2) beweglich ist.

3. Prothesenschaft nach einem der vorstehenden Ansprüche, **dadurch gekennzeichnet, dass** die Halteeinrichtung (17) an der Außenwand des Schaftkörpers (2), vorzugsweise mit ihrem distalen Ende, fixiert ist.

4. Prothesenschaft nach einem der vorstehenden Ansprüche, **dadurch gekennzeichnet, dass** die Halteeinrichtung (17) in Höhe oder parallel einer Schafteintrittsebene (20), die senkrecht zur Stumpfeinführrichtung (22) liegt, am Schaftkörper (2) fixiert ist.

5. Prothesenschaft nach einem der vorstehenden Ansprüche, **dadurch gekennzeichnet, dass** die Halteeinrichtung (17) aus mehreren um den Umfang des Schaftkörpers (2) herum angeordneten Haltebändern (18) oder -seilen besteht.

6. Prothesenschaft nach einem der Ansprüche 1 bis 4, **dadurch gekennzeichnet, dass** die Halteeinrichtung (17) aus einem schlauchartigen Halteorgan besteht, das den Schaftkörper (2) umgibt.

7. Prothesenschaft nach Anspruch 5, **dadurch gekennzeichnet, dass** die Halte-bänder (18) oder -seile jeweils an einem Aufspannelement (13) befestigt sind.

8. Prothesenschaft nach einem der vorhergehenden Ansprüche, **dadurch gekennzeichnet, dass** die Aufspannelemente (13) aus länglichen, biegsamen Führungsstäben oder -leisten bestehen, die mit dem Einführstrumpf (12) fest verbunden oder in Führungslaschen oder -taschen des Einführstrumpfs (12) eingesetzt sind.

9. Prothesenschaft nach einem der vorhergehenden Ansprüche, **dadurch gekennzeichnet, dass** eine Mehrzahl von Aufspannelementen (13) über den Umfang des Einführstrumpfs (12) herum mit einem zumindest annähernd gleichen gegenseitigen Abstand angeordnet sind.

10. Prothesenschaft nach einem der vorhergehenden Ansprüche, **dadurch gekennzeichnet, dass** sich die Aufspannelemente (13) vom Boden (26) des Einführinlays (11) bis zu dessen proximalem Randbereich (16) erstrecken.

11. Prothesenschaft nach einem der vorhergehenden Ansprüche, **dadurch gekennzeichnet, dass** das Einführinlay (11) in seiner äußeren Position zumindest überwiegend umgestülpt ist, wobei sich der Boden (26) des Einführinlays (11) außerhalb des Schaftkörpers (2) oder auf gleicher Höhe wie der am weitesten proximal vorstehende Randbereich des Schaftkörpers (2) befindet.

12. Prothesenschaft nach einem der vorhergehenden Ansprüche, **dadurch gekennzeichnet, dass** zwischen dem sich in seiner inneren Position befindenden Einführinlay (11) und dem Schaftkörper (2) ein flexibles Schaftinlay (5) angeordnet ist, das zusammen mit dem Schaftkörper (2) oder allein einen fluiddichten Fluiddruckraum (9) begrenzt, durch Erzeugen eines Überdrucks im Fluiddruckraum (9) in proximaler Richtung ausstülpbar ist und einen relativ zum Schaftkörper (2) verschiebbaren Boden (10) aufweist.

13. Prothesenschaft nach Anspruch 12, **dadurch gekennzeichnet, dass** das Einführinlay (11) im Bereich des Bodens (10) mit dem Schaftinlay (5) verbunden ist.

14. Prothesenschaft nach einem der vorhergehenden Ansprüche, **dadurch gekennzeichnet, dass** die Aufspannelemente (13) aus Kunststoff oder Federstahl bestehen.

15. Prothesenschaft nach einem der Ansprüche 12 bis 14, **dadurch gekennzeichnet, dass** der Boden (10) des Schaftinlays (5) als formstabile Kappe (7) ausgebildet ist.

16. Prothesenschaft nach einem der Ansprüche 12 bis 15, **dadurch gekennzeichnet, dass** das Schaftinlay (5) mit dem Schaftkörper (2) in einer senkrecht zur Stumpfeinführrichtung (22) liegenden Schafteintrittsebene (20) fluiddicht verbunden ist.

17. Prothesenschaft nach einem der Ansprüche 12 bis 16, **dadurch gekennzeichnet, dass** der Fluiddruckraum (9) über einen durch den Schaftkörper (2) hindurchgeführten Fluidkanal (8) mit einer am Schaftkörper (2) befestigten oder getrennt vom Schaftkörper (2) angeordneten Überdruckerzeugungseinrichtung (28) verbunden oder verbindbar ist.

18. Prothesenschaft nach einem der vorstehenden Ansprüche, **gekennzeichnet durch** wenigstens ein Zugelement (15), durch das ein distales Ende des Einführinlays (11) in Richtung auf ein distales Ende des Schaftkörpers (2) bewegbar ist.

## Claims

1. Prosthesis socket (1) with a socket body (2) for receiving an amputation stump (19) and with an insertion aid which facilitates the insertion of the amputation stump (19) into the socket body (2), whereby the insertion aid comprises an insertion inlay (11) which, in the proximal edge area (16) thereof, is connected to a holding device (17) which is fixed on the socket body (2), wherein the insertion inlay (11) has an insertion stocking (12), which is movable between an outer position, in which the insertion inlay (11) is located at least in part, preferably at least for the most part, outside the socket body (2), and an inner position, in which the insertion stocking (12) lines the socket body (2), **characterized in that** the insertion inlay (11) has elastic, pressure-stable stretching elements (13) which extend along the insertion stocking (12), are fixed on the latter and are movable together with the insertion stocking (12).

2. Prosthesis socket as claimed in claim 1, **characterized in that** the holding device (17) is movable relative to the proximal socket edge (3) of the socket body (2).

3. Prosthesis socket as claimed in one of the preceding claims, **characterized in that** the holding device (17) is fixed, preferably by its distal end, on the outer wall of the socket body (2).

4. Prosthesis socket as claimed in one of the preceding claims, **characterized in that** the holding device (17) is fixed on the socket body (2) at the height of or parallel to a socket entry plane (20), which lies perpendicularly with respect to the stump insertion direction (22).

5. Prosthesis socket as claimed in one of the preceding claims, **characterized in that** the holding device (17) is composed of a plurality of holding tapes (18) or cords arranged about the circumference of the socket body (2).

6. Prosthesis socket as claimed in one of claims 1 to 4, **characterized in that** the holding device (17) is composed of a hose-like holding member that surrounds the socket body (2).

7. Prosthesis socket as claimed in claim 5, **characterized in that** the holding tapes (18) or cords are each secured on a stretching element (13).

8. Prosthesis socket as claimed in one of the preceding claims, **characterized in that** the stretching elements (13) are composed of elongate, flexible guide rods or slats, which are firmly connected to the insertion stocking (12) or are fitted in guide tabs or pockets of the insertion stocking (12).

9. Prosthesis socket as claimed in one of the preceding claims, **characterized in that** a plurality of stretching elements (13) are arranged about the circumference of the insertion stocking (12), with an at least approximately identical spacing between them.

10. Prosthesis socket as claimed in one of the preceding claims, **characterized in that** the stretching elements (13) extend from the bottom (26) of the insertion inlay (11) to the proximal edge area (16) of the latter.

11. Prosthesis socket as claimed in one of the preceding claims, **characterized in that** the insertion inlay (11), in its outer position, is at least for the most part inverted, wherein the bottom (26) of the insertion inlay (11) is located outside the socket body (2) or at the same height as the farthest proximally protruding edge area of the socket body (2).

12. Prosthesis socket as claimed in one of the preceding claims, **characterized in that** a flexible socket inlay (5) is arranged between the insertion inlay (11), located in its inner position, and the socket body (2), which flexible socket inlay (5) delimits, on its own or together with the socket body (2), a fluid-tight fluid pressure chamber (9), can be pushed out in the proximal direction by generation of an overpressure in the fluid pressure chamber (9) and has a bottom (10) movable relative to the socket body (2).

13. Prosthesis socket as claimed in claim 12, **characterized in that** the insertion inlay (11) is connected to the socket inlay (5) in the area of the bottom (10).

14. Prosthesis socket as claimed in one of the preceding claims, **characterized in that** the stretching elements (13) are made of plastic or spring steel.

15. Prosthesis socket as claimed in one of claims 12 to 14, **characterized in that** the bottom (10) of the socket inlay (5) is designed as a dimensionally stable cap (7).

16. Prosthesis socket as claimed in one of claims 12 to 15, **characterized in that** the socket inlay (5) is connected in a fluid-tight manner to the socket body (2) in a socket entry plane (20) that lies perpendicularly with respect to the stump insertion direction (22).

17. Prosthesis socket as claimed in one of claims 12 to 16, **characterized in that** the fluid pressure chamber (9) is connected or connectable, via a fluid channel (8) routed through the socket body (2), to an overpressure generator (28) which is secured on the socket body (2) or which is arranged separately from the socket body (2).

18. Prosthesis socket as claimed in one of the preceding claims, **characterized by** at least one tensioning element (15), by means of which a distal end of the insertion inlay (11) is movable in the direction of a distal end of the socket body (2).

## Revendications

1. Emboîture de prothèse (1) comprenant un corps d'emboîture (2) pour la réception d'un moignon d'amputation (19) et un dispositif d'aide à l'introduction, qui facilite l'introduction du moignon d'amputation (19) dans le corps d'emboîture (2), dans laquelle le dispositif d'aide à l'introduction inclut un insert d'introduction (11), lequel est relié dans sa zone de bordure proximale (16) à un moyen de retenue (17), celui-ci étant fixé au corps d'emboîture (2), dans laquelle l'insert d'introduction (11) comprend une chaussette d'introduction (12), laquelle est déplaçable entre une position extérieure, dans laquelle l'insert d'introduction (11) se trouve au moins partiellement et de préférence au moins en majeure partie, à l'extérieur du corps d'emboîture (2), et une position intérieure dans laquelle la chaussette d'introduction (12) recouvre le corps d'emboîture (2),
**caractérisée en ce que** l'insert d'introduction (11) comprend des éléments de tensionnement élastiques (13) stables vis-à-vis de la pression, qui s'étendent le long de la chaussette d'introduction (12), qui sont fixés sur celle-ci, et qui sont déplaçables conjointement avec la chaussette d'introduction (12).

2. Emboîture de prothèse selon la revendication 1, **caractérisée en ce que** le moyen de retenue (17) est déplaçable par rapport à la bordure d'emboîture proximale (3) du corps d'emboîture (2).

3. Emboîture de prothèse selon l'une des revendications précédentes, **caractérisée en ce que** le moyen de retenue (17) est fixé sur la paroi extérieure du corps d'emboîture (2), de préférence avec son extrémité distale.

4. Emboîture de prothèse selon l'une des revendications précédentes, **caractérisée en ce que** le moyen de retenue (17) est fixé sur le corps d'emboîture à la hauteur de ou parallèlement à un plan d'entrée d'emboîture (20), perpendiculaire à la direction d'introduction du moignon (22).

5. Emboîture de prothèse selon l'une des revendications précédentes, **caractérisée en ce que** le moyen de retenue (17) est composé de plusieurs bandes de retenue (18) ou plusieurs câbles de retenue agencé(e)s tout autour de la périphérie du corps d'emboîture (2).

6. Emboîture de prothèse selon l'une des revendications 1 à 4, **caractérisée en ce que** le moyen de retenue (17) est composé d'un organe de retenue semblable à un tuyau souple, qui entoure le corps d'emboîture (2).

7. Emboîture de prothèse selon la revendication 5, **caractérisée en ce que** les bandes de retenues (18) ou les câbles de retenue sont respectivement fixé(e)s sur un élément de tensionnement (13).

8. Emboîture de prothèse selon l'une des revendications précédentes, **caractérisée en ce que** les éléments de tensionnement (13) sont constitués par des barres ou des barrettes de guidage allongées flexibles, qui sont fermement reliées à la chaussette d'introduction (12) ou qui sont introduites dans des languettes de guidage ou des poches de guidage de la chaussette d'introduction (12).

9. Emboîture de prothèse selon l'une des revendications précédentes, **caractérisée en ce qu'**une pluralité d'éléments de tensionnement (13) sont agencés tout autour de la périphérie de la chaussette d'introduction (12) avec un écartement mutuel au moins approximativement égal.

10. Emboîture de prothèse selon l'une des revendications précédentes, **caractérisée en ce que** les éléments de tensionnement (13) s'étendent depuis le fond (26) de l'insert d'introduction (11) jusqu'à sa zone de bordure proximale (16).

11. Emboîture de prothèse selon l'une des revendications précédentes, **caractérisée en ce que** l'insert d'introduction (11) est rebroussé, au moins en majeure partie, dans sa position extérieure, et le fond (26) de l'insert d'introduction (11) se trouve à l'extérieur du corps d'emboîture (2) ou bien à la même hauteur que la zone de bordure qui dépasse le plus loin en direction proximale, du corps d'emboîture (2).

12. Emboîture de prothèse selon l'une des revendications précédentes, **caractérisée en ce que**, entre l'insert introduction (11) qui se trouve dans sa position intérieure et le corps d'emboîture (2), est agencé un insert d'emboîture flexible (5) qui limite conjointement avec le corps d'emboîture (2) ou seul un compartiment à fluide sous pression (9) étanche vis-à-vis des fluides, qui est susceptible d'être retroussé en direction proximale par génération d'une surpression dans le compartiment à fluide sous pression (9), et qui comprend un fond (10) susceptible d'être déplacé par rapport au corps d'emboîture (2).

13. Emboîture de prothèse selon la revendication 12, **caractérisée en ce que** l'insert introduction (11) est relié à l'insert d'emboîture (5) dans la région du fond (10).

14. Emboîture de prothèse selon l'une des revendications précédentes, **caractérisée en ce que** les éléments de tensionnement (13) sont en matière plastique ou en acier à ressort.

15. Emboîture de prothèse selon l'une des revendications 12 à 14, **caractérisée en ce que** le fond (10) de l'insert d'emboîture (5) est réalisé comme un capuchon (7) à forme stable.

16. Emboîture de prothèse selon l'une des revendications 12 à 15, **caractérisée en ce que** l'insert d'emboîture (5) est relié de manière étanche aux fluides avec le corps d'emboîture (2) dans un plan d'entrée d'emboîture (20) situé perpendiculairement à la direction d'introduction du moignon (22).

17. Emboîture de prothèse selon l'une des revendications 12 à 16, **caractérisée en ce que** le compartiment à fluide sous pression (9) est relié ou susceptible d'être relié à un moyen de génération de surpression fixé sur le corps d'emboîture (2) agencé séparément du corps d'emboîture (2), via un canal à fluide (8) passé à travers le corps d'emboîture (2).

18. Emboîture de prothèse selon l'une des revendications précédentes, **caractérisée par** au moins un élément de traction (15), au moyen duquel une extrémité distale de l'insert introduction (11) est déplaçable en direction d'une extrémité distale du corps d'emboîture (2).
